(19) Europäisches Patentamt
European Patent Office
Office européen des brevets

(11) Veröffentlichungsnummer: **0 548 669 B1**

(12) **EUROPÄISCHE PATENTSCHRIFT**

(45) Veröffentlichungstag der Patentschrift: **12.07.95**

(51) Int. Cl.$^{6}$: **C07C 271/08**, C08G 18/80

(21) Anmeldenummer: **92120943.3**

(22) Anmeldetag: **09.12.92**

# (54) Isocyanatocarbonsäuren, ein Verfahren zu ihrer Herstellung und ihrer Verwendung.

(30) Priorität: **20.12.91 DE 4142275**

(43) Veröffentlichungstag der Anmeldung:
**30.06.93 Patentblatt 93/26**

(45) Bekanntmachung des Hinweises auf die Patenterteilung:
**12.07.95 Patentblatt 95/28**

(84) Benannte Vertragsstaaten:
**AT BE CH DE ES FR GB IT LI NL SE**

(56) Entgegenhaltungen:
**EP-A- 0 153 561**
**EP-A- 0 365 098**
**EP-A- 0 443 138**
**DE-A- 3 608 354**
**DE-A- 3 620 821**
**FR-A- 969 550**

(73) Patentinhaber: **BAYER AG**

**D-51368 Leverkusen (DE)**

(72) Erfinder: **Zwiener, Christian, Dr.**
**Hittorfstrasse 9**
**W-5000 Köln 60 (DE)**
Erfinder: **Rettig, Rainer, Dr., Shukuyawa Sunny Gard, App 303**
**Zur Linde 27**
**W-5067 Kürten-Engeldorf (DE)**
Erfinder: **Nachtkamp, Klaus, Dr.**
**Silcherstrasse 13**
**W-4000 Düsseldorf 13 (DE)**
Erfinder: **Pedain, Josef, Dr.**
**Haferkamp 6**
**W-5000 Köln 80 (DE)**
Erfinder: **Arlt, Dieter, Professor Dr.**
**Rybnikerstrasse 2**
**W-5000 Köln 80 (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue, lagerstabile, an tertiäre Kohlenstoffatome gebundene Isocyanatgruppen und Carboxylgruppen in gegebenenfalls zumindest teilweise neutralisierter Form aufweisende Isocyanatocarbonsäuren, ein Verfahren zu ihrer Herstellung, sowie ihre Verwendung als Vernetzer für wäßrige Beschichtungsmittel.

In den letzten Jahren stieg die Bedeutung wäßriger Lacke und Beschichtungsmittel aufgrund immer strengerer Emmissionsrichtlinien bezüglich der bei der Lackapplikation freiwerdenden Lösemittel stark an. Obwohl inzwischen bereits für viele Anwendungsbereiche wäßrige Lacksysteme zur Verfügung stehen, können diese das hohe Qualitätsniveau konventioneller, lösemittelhaltiger Lacke hinsichtlich Lösemittel- und Chemikalienbeständigkeit oder gegenüber mechanischer Belastbarkeit oftmals nur durch eine chemische Nachvernetzung während der Filmbildung erreichen.

Versuche, eine Nachvernetzung wäßriger Lacke mit Hilfe freie Isocyanatgruppen tragender Verbindungen zu erreichen, d.h. das auf dem Gebiet lösemittelhaltiger Beschichtungen bewährte Konzept der Polyurethanreaktivlacke auf wäßrige Systeme zu übertragen, wurden bislang nur wenige unternommen.

Die DE-OS 2 708 442 beschreibt den Zusatz monomerer organischer Diisocyanate zur Verbesserung des Eigenschaftsbildes wäßriger Polyurethandispersionen.

Nach der DE-OS 3 529 249 lassen sich organische Polyisocyanate zur Erhöhung der Lösemittel- und Chemikalienbeständigkeit sowie zur Verbesserung der Verschleißeigenschaften von Beschichtungen auf Basis von in Wasser dispergierten Homo- und Copolymerisaten einsetzen. Die beschriebenen positiven Effekte auf die Lackeigenschaften lassen sich auf eine durch Reaktion der Isocyanate mit Wasser gebildete "Harnstoffhülle" um die dispergierten Polymerteilchen zurückführen. Die als Zusatzmittel eingesetzten Polyisocyanate wirken somit nicht als Vernetzungsmittel für in Wasser dispergierte Kunststoffe oder Kunststoffvorläufer mit gegenüber Isocyanatgruppen reaktionsfähigen Gruppen.

Die Herstellung eines wäßrigen Polyurethanreaktivsystems aus ausgewählten, Emulgatorwirkung aufweisenden, wäßrigen Polyhydroxylverbindungen und niedrigviskosen freien Polyisocyanaten ist Gegenstand der DE-OS 3 829 587. Dabei werden Lackfilme erhalten, deren Eigenschaftsbild dem typischer, lösemittelhaltiger Zweikomponentenlacke entspricht. Aufgrund der allgemein schlechten Dispergierbarkeit organischer Polyisocyanate in Wasser ist dieses Verfahren jedoch auf spezielle Polyoldispersionen beschränkt. Zur Übertragung auf beliebige wäßrige, gegenüber Isocyanaten reaktive Gruppen tragende Bindemittel bedarf es hydrophilierter, selbstdispergierbarer Polisocyanatkomponenten.

Aromatische, hydrophil modifizierte Polyisocyanate, wie sie z.B. in der DE-OS 2 359 613 und der EP-A-61 628 beschrieben werden, sind aufgrund ihrer hohen Reaktivität gegenüber Wasser und der damit verbundenen Kohlendioxid-Entwicklung zum Einsatz in wäßrigen Lacksystemen nicht geeignet. Sie finden bevorzugt bei der Schaumstoff-Herstellung und auf dem Klebstoffsektor Verwendung. Isocyanat-funktionelle Vernetzer für wäßrige Beschichtungssysteme sind ausschließlich auf Basis der weniger reaktiven (cyclo)-aliphatischen Polyisocyanate herstellbar.

In der EP-A-0 206 059 werden hydrophil modifizierte, aliphatische Polyisocyanate als Zusatzmittel für wäßrige Klebstoffe beschrieben. Die Emulgierbarkeit dieser Polyisocyanate wird durch den Einbau Ethylenoxideinheiten aufweisender Polyetherketten erreicht. Für eine Anwendung auf dem Lacksektor sind derart hydrophilierte Polyisocyanate aufgrund der durch den relativ hohen Polyethergehalt verursachten, bleibenden Hydrophilie der Beschichtung jedoch weniger geeignet.

In der DE-OS 4 001 783 werden Carboxylgruppen enthaltende Polyisocyanatgemische beschrieben, die als erfindungswesentliches Kriterium Uretdiongruppen aufweisen. Diese Produkte werden u.a. als Bindemittel für Überzugsmittel verwendet. Da die Bereitstellung von Uretdiongruppen enthaltenden Polyisocyanaten einen separaten Verfahrensschritt, nämlich die Dimerisierung der Ausgangsisocyanate erfordert, handelt es sich hier um ein relativ aufwendiges Verfahren. Ein weiterer Nachteil, der prinzipiell allen Verfahren zur Herstellung Isocyanatgruppen enthaltender Produkte auf Basis der aus der Polyurethanlackchemie bekannten Diisocyanate, wie z.B. Hexamethylendiisocyanat und Isophorondiisocyanat, zu eigen ist, liegt darin, daß nach der eigentlichen Herstellung ein aus arbeitshygienischer Sicht zu hoher Gehalt an Restmonomeren vorliegt, der anschließend mit aufwendigen Verfahren, wie z.B. der Dünnschichtdestillation, entfernt werden muß.

Es war daher die der Erfindung zugrunde liegende Aufgabe, Verbindungen zur Verfügung zu stellen, die sowohl Isocyanatgruppen als auch Carboxylgruppen tragen, die über einen längeren Zeitraum keine nennenswerte Reaktion zwischen den Isocyanat- und Carboxylgruppen zeigen, also lagerstabil sind, die nach Neutralisation mit Basen in Wasser löslich bzw. dispergierbar sind, die in wäßriger Phase eine Topfzeit von mehreren Stunden aufweisen und zur Vernetzung von wäßrigen Lackbindemitteln verwendbar sind. Außerdem sollte das Verfahren zur Herstellung der neuen Verbindungen zu Verfahrensprodukten mit

einem Gehalt an monomerem Diisocyanat von unter 2 Gew.-% führen, ohne daß hierzu eine aufwendige Nachbehandlung erforderlich ist.

Diese Aufgabe konnte mit der Bereitstellung der nachstehend näher beschriebenen erfindungsgemäßen Isocyanatocarbonsäuren bzw. dem Verfahren zu ihrer Herstellung gelöst werden.

Gegenstand der Erfindung sind Isocyanatocarbonsäuren, gekennzeichnet durch

a) einen Gehalt an, an (cyclo)aliphatische tertiäre Kohlenstoffatome gebundenen, Isocyanatgruppen von 1 bis 30 Gew.-% und

b) einen Gehalt an, gegebenenfalls zumindest teilweise in der Salzform vorliegenden, Carboxylgruppen von 0,5 bis 500 Milliäquivalenten pro 100 g.

Gegenstand der Erfindung ist auch ein Verfahren zur Herstellung dieser Isocyanatocarbonsäuren, welches dadurch gekennzeichnet ist, daß man

A) Diisocyanate des Molekulargewichtsbereichs 168 bis 300 mit einer an ein primäres aliphatisches Kohlenstoffatom gebundenen Isocyanatgruppe und einer an ein tertiäres (cyclo)aliphatisches Kohlenstoffatom gebundenen Isocyanatgruppe, sowie gegebenenfalls

B) bis zu 25 NCO-Äquivalentprozent, bezogen auf die Gesamtmenge der Komponenten A) und B), an anderen Diisocyanaten des Molekulargewichtsbereichs 168 bis 300, mit (cyclo)aliphatisch gebundenen Isocyanatgruppen

mit

C) 2,2-Bis(hydroxymethyl)alkansäuren der Formel

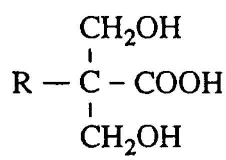

in welcher

R für Wasserstoff, einen Hydroxymethylrest oder einen Alkylrest mit bis zu 20 Kohlenstoffatomen steht,

sowie gegebenenfalls

D) sonstigen organischen Polyhydroxylverbindungen

unter Einhaltung eines NCO/OH-Äquivalentverhälmisses von 1,6:1 bis 2:1, gegebenenfalls unter vorhergehender oder anschließender zumindest teilweiser Neutralisation der Carboxylgruppen der Komponente C) mit einer Base, zur Reaktion bringt, wobei im übrigen Art und Mengenverhältnisse der Ausgangskomponenten so gewählt werden, daß die resultierenden Isocyanatocarbonsäuren den oben genannten Angaben entsprechen.

Gegenstand der Erfindung ist schließlich auch die Verwendung der neuen Isocyanatocarbonsäuren als Vernetzer für wäßrige Beschichtungsmittel auf Basis von in Wasser gelöst und/oder dispergiert vorliegenden Kunststoffen oder Kunststoffvorläufern.

Geeignete Diisocyanate A) sind beliebige Diisocyanate des Molekulargewichtsbereichs 168 bis 300, die eine an ein tertiäres (cyclo)aliphatisches Kohlenstoffatom gebundene und eine an ein primäres aliphatisches Kohlenstoffatom gebundene Isocyanatgruppe aufweisen. In Betracht kommen beispielsweise Diisocyanate der Formel

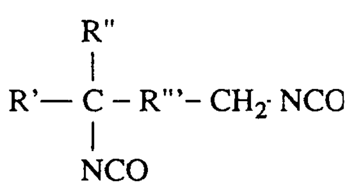

für welche

R' und R'' für gleiche oder verschiedene Reste stehen und Alkylgruppen mit 1 bis 4 Kohlenstoffatomen bedeuten und

R''' für einen zweiwertigen, gegebenenfalls verzweigten, gesättigten aliphatischen Kohlenwasserstoffrest mit 2 bis 9 Kohlenstoffatomen steht.

Derartige Diisocyanate bzw. ihre Herstellung sind beispielsweise in DE-OS 3 608 354 oder DE-OS 3 620 821 beschrieben. Bevorzugte derartige Diisocyanate sind solche, für welche R' und R'' jeweils für Methylreste und R''' für zweiwertige, lineare aliphatische Kohlenwasserstoffreste mit 2 bis 5 Kohlenstoffatomen stehen. Typische Vertreter sind beispielsweise 1,4-Diisocyanato-4-methylpentan, 1,5-Diisocyanato-5-methylhexan. 1,6-Diisocyanato-6-methylheptan, 1,5-Diisocyanato-2,2,5-trimethylhexan oder 1,7-Diisocyanato-3,7-dimethyloctan.

Bevorzugt werden jedoch als Ausgangskomponente A) aliphatisch-cycloaliphatische Diisocyanate der allgemeinen Formel

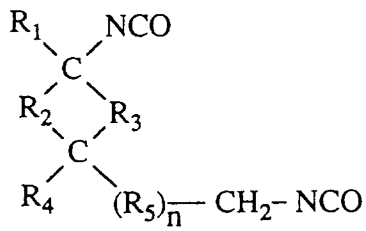

verwendet, für welche

- $R_1$ für einen Alkylrest mit 1 bis 4 Kohlenstoffatomen, vorzugsweise einen Methylrest steht,
- $R_2$ und $R_3$ für gleiche oder verschiedene zweiwertige, gesättigte aliphatische Kohlenwasserstoffreste mit 1 bis 4 Kohlenstoffatomen, vorzugsweise 1 bis 3 Kohlenstoffatomen stehen, wobei die Summe der Kohlenstoffatome dieser Reste vorzugsweise 3 bis 6, insbesondere 4 oder 5 beträgt,
- $R_4$ für Wasserstoff oder für einen Alkylrest mit 1 bis 4 Kohlenstoffatomen, vorzugsweise für Wasserstoff oder einen Methylrest steht,
- $R_5$ für einen zweiwertigen, gesättigten aliphatischen Kohlenwasserstoffrest mit 1 bis 4, insbesondere 1 bis 3, Kohlenstoffatomen steht und
- n für 0 oder 1 steht.

Als Ausgangskomponente A) besonders bevorzugte derartige aliphatisch-cycloaliphatische Diisocyanate sind z.B. 1-Isocyanato-1-methyl-4(3)-isocyanatomethylcyclohexan, welches im allgemeinen als Gemisch der 4- und 3-Isocyanatomethyllsomeren vorliegt, 1-Isocyanato-1-methyl-4-(4-isocyanatobut-2-yl)-cyclohexan, 1-Isocyanato-1,2,2-trimethyl-3-(2-isocyanatoethyl)-cyclopentan oder 1-Isocyanato-1,4(3)-dimethyl-4(3)-isocyanatomethyl-cyclohexan, welches im allgemeinen in Form eines 4-Methyl-4-isocyanatomethyl- und 3-Methyl-3-isocyanatomethyl-Isomerengemischs vorliegt. Geeignet sind jedoch auch z.B. 1-Isocyanato-1-n-butyl-3-(4-isocyanatobut-1-yl)-cyclohexan oder 1-Isocyanato-1,2-dimethyl-3-ethyl-3-isocyanatomethyl-cyclopentan.

Die Herstellung derartiger aliphatisch-cycloaliphatischer Diisocyanate ist beispielsweise in EP-A-0 153 561 beschrieben. Beliebige Gemische der beispielhaft genannten Diisocyanate der zuletzt genannten allgemeinen Formeln können selbstverständlich ebenfalls als Komponente A) beim erfindungsgemäßen Verfahren verwendet werden.

Die Diisocyanate A) können gegebenenfalls zusammen mit anderen Diisocyanaten B) zum Einsatz gelangen, wobei die Diisocyanate B) maximal in einer Menge von 25 NCO-Äquivalentprozent, bezogen auf die Gesamtmenge der Diisocyanate A) und B) verwendet werden.

Bei den gegebenenfalls mitzuverwendenden Diisocyanaten B) handelt es sich um solche mit aliphatisch und/oder cycloaliphatisch gebundenen Isocyanatgruppen des Molekulargewichtsbereichs 168 bis 300, welche keine an tertiäre Kohlenstoffatome gebundene Isocyanatgruppen aufweisen, wie beispielsweise von Hexamethylendiisocyanat, 1-Isocyanato-3,3,5-trimethyl-5-isocyanatomethylcyclohexan und/oder von 4,4'-Diisocyanato-dicyclohexylmethan. Die Mitverwendung derartiger Diisocyanate B) ist allerdings nicht bevorzugt.

Bei der Komponente C) handelt es sich um 2,2-Bis(hydroxymethyl)alkansäuren der Formel

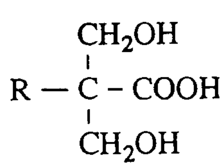

wobei

R für Wasserstoff, einen Hydroxymethylrest oder einen Alkylrest mit bis zu 20 Kohlenstoffatomen, vorzugsweise mit bis zu acht Kohlenstoffatomen steht. Besonders bevorzugt werden 2,2-Bis-(hydroxymethyl)essigsäure, 2,2,2-Tris(hydroxymethyl)essigsäure, 2,2-Bis(hydroxymethyl)-butansäure und 2,2-Bis(hydroxymethyl)pentansäure.Ganz besonders bevorzugt wird 2,2-Bis-(hydroxymethyl)propionsäure.

Bei der gegebenenfalls mitzuverwendenden Komponente D) handelt es sich beispielsweise um D1) niedermolekulare aliphatische Polyole des Molekulargewichtsbereichs 62 bis 799 und/oder um D2) höhermolekulare Polyhydroxylverbindungen eines dampfdruck bzw. membranosmometrisch bestimmten Molekulargewichts Mn von 800 bis 12.000, vorzugsweise 800 bis 5 000, und/oder um D3) hydrophile Gruppen aufweisende ein- oder mehrwertige Alkohole. Unter diesen Hydroxylgruppen aufweisenden Verbindungen D) sind die niedermolekularen, mehrwertigen Alkoholen D1) bevorzugt.

Bei den niedermolekularen, mehrwertigen Alkoholen handelt es sich um aliphatische Polyole des genannten Molekulargewichtsbereichs, vorzugsweise des Molekulargewichtsbereichs 62 bis 200, wie beispielsweise Ethandiol, 1,2- und 1,3-Propandiol, 1,3-, 2,3- und 1,4-Butandiol, 1,6-Hexandiol, 1,8-Octandiol, Neopentylglykol, 1,4-Bis(hydroxymethyl)cyclohexan, 2-Methyl-1,3-propandiol, 2,2,4-Trimethyl-1,3-pentandiol, 2-Ethyl-1,3-hexandiol, Perhydrobisphenol A, Glycerin, Trimethylolpropan, 1,2,6-Hexantriol, niedermolekulare Hydroxylgruppen aufweisende Ester derartiger Polyole mit Dicarbonsäuren der nachstehend beispielhaft genannten Art oder niedermolekulare Ethoxylierungs- und/oder Propoxylierungsprodukte derartiger einfacher Polyole oder beliebige Gemische derartiger mehrwertiger modifizierter oder nichtmodifizierter Alkohole.

Bei den höhermolekularen Polyhydroxylverbindungen D2) handelt es sich um die höhermolekularen Polyhydroxylverbindungen der aus der Polyurethanchemie an sich bekannten Art. Diese Polyhydroxylverbindungen tragen pro Molekül mindestens zwei Hydroxylgruppen und weisen im allgemeinen einen Hydroxylgruppengehalt von 0,3 bis 17, vorzugsweise 0,9 bis 6 Gew.-%, auf.

Höhermolekulare Polyhydroxylverbindungen sind beispielsweise die den gemachten Angaben entsprechenden Polyester-Polyole auf Basis von niedermolekularen einfachen Alkoholen der bereits beispielhaft genannten Art und mehrbasischen Carbonsäuren wie beispielsweise Adipinsäure, Sebacinsäure, Phthalsäure, Isophthalsäure, Tetrahydrophthalsäure, Hexahydrophthalsäure, Maleinsäure, den Anhydriden derartiger Säuren oder beliebigen Gemischen derartiger Säuren bzw. Säureanhydride. Auch den obigen Angaben entsprechende, Hydroxylgruppen aufweisende Polylactone, insbesondere Poly-$\epsilon$-caprolactone sind geeignet.

Ebenfalls als Komponente D2) geeignet sind die, obigen Ausführungen entsprechenden, Polyether-Polyole, wie sie in an sich bekannter weise durch Alkoxylierung von geeigneten Startermoleküle sind beispielsweise die oben bereits genannten einfachen Polyole, Wasser, organische Polyamine mit mindestens zwei N-H-Bindungen oder beliebige Gemische derartiger Startermoleküle. Für die Alkoxylierungsreaktion geeignete Alkylenoxide sind insbesondere Ethylenoxid und/oder Propylenoxid, die in beliebiger Reihenfolge oder auch im Gemisch bei der Alkoxylierungsreaktion eingesetzt werden können.

Ferner als Komponente D2) geeignet sind die, obigen Ausführungen entsprechenden, Hydroxylgruppen aufweisenden Polycarbonate, wie sie beispielsweise durch Umsetzung von einfachen Diolen der oben bereits beispielhaft genannten Art mit Diarylcarbonaten, beispielsweise Diphenylcarbonat oder Phosgen hergestellt werden können.

Ebenfalls als Komponente D2) geeignet sind die, obigen Ausführungen entsprechenden, Polyhydroxypolyacrylate der bekannten Art.

Es handelt sich bei diesen Verbindungen um Copolymerisate von Hydroxylgruppen aufweisenden olefinischen Monomeren mit Hydroxylgruppen-freien olefinischen Monomeren. Beispiele für geeignete Monomere sind Vinyl- bzw. Vinylidenmonomere wie z.B. Styrol, $\alpha$-Methylstyrol, o- bzw. p-Chlorstyrol, o-, m- oder p-Methylstyrol, p-tert.-Butylstyrol, Acrylsäure, (Meth)Acrylnitril, Acryl- und Methacrylsäureester mit 1 bis 8 Kohlenstoffatomen in der Alkoholkomponente wie beispielsweise Ethylacrylat, Methylacrylat, n- bzw. Isopropylacrylat, n-Butylacrylat, 2-Ethylhexylacrylat, 2-Ethylhexylmethacrylat, Isooctylacrylat, Methylmethacrylat, Ethylmethacrylat, Butylmethacrylat, Isooctylmethacrylat; Diester der Fumarsäure, Itaconsäure, Maleinsäure mit 4 bis 8 Kohlenstoffaromen in der Alkoholkomponente, (Meth)Acrylsäureamid, Vinylester von Alkanmonocarbonsäuren mit 2 bis 5 Kohlenstoffatomen wie Vinylacetat oder Vinylpropionat und Hydroxyalkylester der Acrylsäure oder Methacrylsäure mit 2 bis 4 Kohlenstoffatomen im Hydroxyalkylrest wie z.B. 2-Hydroxyethyl-, 2-Hydroxypropyl-, 4-Hydroxybutyl, Trimethylolpropanmono- oder Pentaerythritmono-acrylat oder -methacrylat. Beliebige Gemische der beispielhaft genannten Monomeren können bei der Herstellung der hydroxyfunktionellen Polyacrylate selbstverständlich auch eingesetzt werden.

Geeignete Hydroxylverbindungen D3) sind beispielsweise hydrophile Seitenketten aufweisende Diole der in der US-PS 4 190 566 genannten Art oder hydrophile einwertige Polyetheralkohole der in der US-PS 4 237 264 genannten Art. Selbstverständlich ist es auch möglich, andere Polyolkomponenten mit hydrophilen

bzw. potentiell hydrophilen Gruppen, wie z.B. Sulfonatgruppen als Komponente D3) oder als Teil der Komponente D3) einzusetzen.

Die erfindungsgemäßen Isocyanatocarbonsäuren weisen einen Gehalt an, an (cyclo)aliphatische tertiäre Kohlenstoffatome gebundenen Isocyanatgruppen von 1 bis 30, vorzugsweise 2 bis 20 Gew.-% und einen Gehalt an, gegebenenfalls zumindest teilweise neutralisiert vorliegenden, Carboxylgruppen von 0,5 bis 500, vorzugsweise 5,0 bis 300 und insbesondere 10 bis 100 Milliäquivalenten pro 100 g Feststoff auf. Die NCO-Funktionalität der erfindungsgemäßen Verbindungen liegt im allgemeinen bei mindestens 2,0, vorzugsweise bei 2,0 bis 4,5. Der Gehalt der Isocyanatocarbonsäuren an innerhalb von Polyetherketten eingebauten Ethylenoxideinheiten ($-CH_2-CH_2-O-$) liegt bei 0 bis 30, vorzugsweise bei 0 bis 20 und besonders bevorzugt bei 0 %. Derartige Polyetherketten liegen in den erfindungsgemäßen Verbindungen dann vor, wenn eine entsprechende Komponente D3) bei der Durchführung des erfindungsgemäßen Verfahrens mitverwendet worden ist.

Bei der Durchführung des erfindungsgemäßen Verfahrens werden die Ausgangskomponenten A) und gegebenenfalls B) sowie C) und gegebenenfalls D) in solchen Mengen eingesetzt, daß ein Äquivalentverhältnis zwischen Isocyanatgruppen zu Hydroxylgruppen von 1,4:1 bis 2:1, vorzugsweise 1,6:1 bis 2:1, vorliegt, mit der Maßgabe, daß im Falle der Mitverwendung von Diisocyanaten B) ohne tertiär gebundene Isocyanatgruppen das Äquivalentverhältnis, bezogen auf nichttertiär gebundene Isocyanatgruppen der Komponenten A) und B) sowie die Hydroxylgruppen der Komponenten C) und D) bei maximal 1:1 liegt. Im übrigen werden Art und Mengenverhältnisse der Ausgangskomponenten im Rahmen der gemachten Offenbarung so gewählt, daß als Verfahrensprodukte Isocyanatocarbonsäuren mit den genannten Eigenschaften resultieren.

Das erfindungsgemäße Verfahren kann sowohl in der Schmelze auch in Gegenwart von inerten Lösungsmitteln durchgeführt werden. Vorzugsweise wird das Verfahren in Gegenwart von gegenüber Isocyanatgruppen inerten Lösungsmitteln durchgeführt, wobei man bevorzugt bis zu 50 Gew.-%, besonders bevorzugt bis zu 40 Gew.-%, Lösungsmittel, bezogen auf Lösung, verwendet.

Brauchbare inerte Lösungsmittel sind z.B. Ethylacetat, Butylacetat, Ethylenglykolmonomethyl- oder -ethyletheracetat, Butyldiglykolacetat, Diethylenglykoldimethylether, 1-Methoxypropyl-2-acetat, 2-Butanon oder deren Gemische.

Zur Durchführung des erfindungsgemäßen Verfahrens kann man beispielsweise so vorgehen, daß man entweder alle Ausgangskomponenten gemeinsam vorlegt, oder die Diisocyanate A) und gegebenenfalls B) gemeinsam mit der Komponente C) vorlegt, homogen verrührt und gegebenenfalls die Komponenten D), u.U. aufgeschmolzen, über einen Tropftrichter zudosiert, wobei man die Reaktion im Temperaturbereich von 20 bis 150 °C, bevorzugt 40 bis 120 °C und besonders bevorzugt von 40 bis 100 °C, ablaufen läßt. Die Reaktion ist beendet, wenn der aus der Stöchiometrie der Ausgangsmaterialien errechnete theoretische NCO-Gehalt erreicht oder geringfügig unterschritten ist.

Der Restmonomerengehalt der so erhaltenen erfindungsgemäßen Verfahrensprodukte liegt im allgemeinen ohne separat durchgeführte Monomerenentfernung unter 2 Gew.-%, vorzugsweise unter 1 Gew.-%. Falls Diisocyanate B) ohne tertiär gebundene Isocyanatgruppen im Rahmen der gemachten Ausführungen mitverwendet werden, werden diese zu mindestens 99,9 % in die Verfahrensprodukte eingebaut, so daß der Gehalt der Verfahrensprodukte an freien, nicht gebundenen Diisocyanaten B) im allgemeinen unter 0,1 Gew.-% liegt.

Die erfindungsgemäßen Isocyanatocarbonsäuren können in lösungsmittelfreier Form, je nach Zusammensetzung, als Flüssigkeiten oder Feststoffe vorliegen. Die Schmelzpunkte der festen Verbindungen können bei bis zu 200 °C liegen. Der Aggregatzustand und der Schmelzpunkt der lösungsmittelfreien Produkte sind durch die Wahl der Ausgangsmaterialien und ihr stöchiometrisches Verhältnis beeinflußbar.

Die Isocyanatocarbonsäuren stellen bei Umgebungstemperatur lagerstabile Verbindungen dar, d.h., daß auch nach längerer Lagerung, z.B. über ein Jahr keine nennenswerte Reaktion zwischen den tertiären Isocyanatgruppen und den Carboxylgruppen stattfindet.

Die erfindungsgemäßen Isocyanatocarbonsäuren stellen wertvolle Vorprodukte zum Aufbau hochmolekularer Polyurethane in wäßrigem Medium dar. Hierzu können z.B. in Wasser gelöste oder dispergierte, Hydroxylgruppen enthaltende Polymere oder Semipolymere mit den zumindest teilweise neutralisierten und in Wasser gelösten oder dispergierten Isocyanatocarbonsäuren im Sinne von wäßrigen Zweikomponenten-Systemen zur Umsetzung gebracht werden.

Besonders bevorzugt werden die erfindungsgemäßen Isocyanatocarbonsäuren, in zumindest teilweise neutralisierter Form, als Vernetzer für wäßrige Beschichtungsmittel auf Basis von in Wasser löslichen und/oder dispergierten Kunststoffen oder Kunststoffvorläufern als Bindemittel verwendet.

Für beide Anwendungen ist eine zumindest teilweise Überführung der Carboxylgruppen in die Salzform durch Neutralisation mit einer geeigneten Base erforderlich, um die Wasserlöslichkeit bzw. -dispergierbarkeit der Isocyanatocarbonsäuren zu gewährleisten. Besonders gut geeignete Basen sind insbesondere tertiäre Amine wie beispielsweise Triethylamin, N-Methylpyrrolidin, N-Methylpiperidin oder N-Methylmorpholin oder gegenüber Isocyanatgruppen reaktionsfähige tertiäre Amine, insbesondere Aminoalkohole wie beispielsweise Triethanolamin, N-Methyldiethanolamin, 2-(N,N-Dimethylamino)-isopropanol oder N,N-Dimethylethanolamin.

Die zumindest teilweise Neutralisation der Carboxylgruppen erfolgt im allgemeinen im Anschluß an die Durchführung der erfindungsgemäßen Additionsreaktion. Grundsätzlich möglich wäre jedoch auch die Verwendung von zumindest teilweise neutralisierten Aufbaukomponenten C) als Ausgangsmaterial bei der Herstellung der Isocyanatocarbonsäuren.

Für den Fall, daß die Isocyanatocarbonsäuren in flüssiger Form, - lösungsmittelfrei oder als Lösung - vorliegen, können diese in das die gewählte Menge Amin enthaltende Wasser eingerührt werden, oder das aminhaltige Wasser kann in die flüssigen Polyisocyanatocarbonsäuren eingerührt werden. Es ist selbstverständlich auch möglich, die flüssigen Isocyanatocarbonsäuren zunächst mit dem Amin teilweise oder ganz zu neutralisieren und dann in Wasser einzubringen bzw. das Wasser in die neutralisierten Isocyanatocarbonsäuren einzutragen. Liegen die erfindungsgemäßen Isocyanatocarbonsäruen als Feststoff vor, so ist es am zweckmäßigsten, diese in gemahlener Form in das die gewünschte Menge Neutralisationsamin enthaltende Wasser einzurühren.

Die Frage, ob bei diesen Verfahrensweisen wäßrige Lösungen oder Dispersionen der erfindungsgemäßen Isocyanatocarbonsäuren entstehen, hängt selbstverständlich von der Art und den Mengenverhältnissen der zur Herstellung der Isocyanatocarbonsäuren eingesetzten Ausgangsmaterialien und insbesondere vom Gehalt an hydrophilen Zentren ab. Auf jeden Fall ist es eine der wichtigsten Eigenschaften der erfindungsgemäßen Isocyanatocarbonsäuren, daß sie im wäßrigen Milieu über einen längeren Zeitraum weitgehend unverändert löslich bzw. dispergierbar sind, da die teriär gebundenen Isocyanatgruppen nur sehr langsam mit dem Wasser abreagieren. Die die erfindungsgemäßen Isocyanatocarbonsäuren enthaltenden wäßrigen Lösungen bzw. Dispersionen weisen im allgemeinen Topfzeiten von mehreren Stunden auf.

Die Vereinigung der erfindungsgemäßen Isocyanatocarbonsäuren mit den zu vernetzenden wäßrigen Beschichtungsbindemitteln kann z.B. durch einfaches Verrühren der nach einer der oben beschriebenen Methoden hergestellten wäßrigen Lösungen bzw. Dispersionen der erfindungsgemäßen Isocyanatocarbonsäuren mit den zu vernetzenden wäßrigen Lack- bzw. Beschichtungsbindemitteln erfolgen.

Es ist auch möglich, die erfindungsgemäßen Isocyanatocarbonsäuren in die das gewählte Neutralisationsamin und gegebenenfalls noch weiteres Wasser enthaltenden zu vernetzenden wäßrigen Beschichtungsbindemittel einzurühren.

Für die Vernetzung mit erfindungsgemäßen Isocyanatocarbonsäuren geeignete wäßrige Beschichtungsmittel sind insbesondere solche, die als Bindemittel Kunststoffe bzw. Kunststoffvorläufer, ausgewählt aus der Gruppe bestehend aus (i) in Wasser dispergierte Polyurethanen, die aufgrund der in den Urethangruppen vorliegenden aktiven Wasserstoffatome mit Polyisocyanaten vernetzbar sind, (ii) in Wasser gelösten oder dispergierten Hydroxylgruppen aufweisenden Polyacrylaten, insbesondere solchen des Molekulargewichtsbereichs 1 000 bis 10 000, die mit organischen Polyisocyanaten als Vernetzer wertvolle Zweikomponenten-Bindemittel darstellen und (iii) in Wasser dispergierte, gegebenenfalls Urethan-modifizierte, Hydroxylgruppen aufweisende Polyesterharze, der aus der Polyester- und Alkydharzchemie an sich bekannten Art.

Den mit erfindungsgemäßen Isocyanatocarbonsäuren formulierten Beschichtungs-Systemen können selbstverständlich die üblichen Hilfs- und Zusatzmittel zugesetzt werden.

Die Applikation dieser zweikomponentigen wäßrigen Beschichtungsmittel kann nach an sich bekannten Methoden, beispielsweise durch Spritzen, Streichen, Tauchen, Fluten oder mit Hilfe von Walzen oder Rakeln auf beliebige Substrate ein- oder mehrschichtig erfolgen.

Die nachfolgenden Beispiele sollen die Erfindung näher erläutern. Alle Prozentangaben und alle Angaben in "Teilen" beziehen sich auf das Gewicht.

**Ausgangsmaterialien A:**

Diisocyanat I

1-Isocyanato-1-methyl-4(3)-isocyanatomethylcyclohexan (Beispiel 1 der DE-OS 3 402 623)

Diisocyanat II

1-Isocyanato-1-methyl-4(4-isocyanatobut-2-yl)-cyclohexan (Beispiel 2 der DE-OS 3 402 623)

**Ausgangsmaterialien D:**

Polyester

Mittleres Molekulargewicht 840, hergestellt aus Adipinsäure und 1,6-Hexandiol Hydroxylgruppengehalt: 4 %.

Polyether I

Linearer, auf Diethylenglykol-mono-n-butylether (="Butyldiglykol") gestarteter Ethylenoxid/Propylenoxid-Mischether, monofunktionell
Molekulargewicht: ca. 2 250
Hydroxylgruppengehalt: 0,75 %.

Polyether II

Tegomer® D-3123 (Th. Goldschmidt AG, Essen), Ethylenoxid/Propylenoxid-Mischether, difunktionell
Molekulargewicht: ca. 1 180
Hydroxylgruppengehalt: 2,9 %.

Dispersion I

Wäßrige Polyester-Dispersion mit einem Festkörpergehalt von 55 % und einer OH-Zahl von ca. 40, hergestellt aus:
7,5 % Trimethylolpropan
15,4 % Hexandiol-1,6
26,4 % Phthalsäureanhydrid
5,6 % Teträhydrophthalsäureanhydrid
3,8 % Dimethylethanolamin
14,7 % Butylglykol
6,7 % i-Butanol
19,8 % Wasser

Dispersion II

Wäßrige Polyester-Dispersion mit einem Festkörpergehalt von 67 % und einer OH-Zahl von ca. 67, hergestellt aus:
25,4 % Hexandiol-1,6
6,7 % Trimethylolpropan
27,8 % Phthalsäureanhydrid
7,1 % Trimellithsäureanhydrid
13,3 % Butyldiglykol
5,2 % Dimethylethanolamin
14,5 % Wasser

Dispersion III

Wäßrige Dispersion eines Hydroxylgruppen enthaltenden Polyester-Polyurethanharzesmit einem Festkörpergehalt von 46,5 % und einer OH-Zahl von ca. 33, hergestellt aus:
1,2 % Isononansäure
5,8 % Trimethylolpropan
7,2 % Hexandiol-1,6
4,0 % Cyclohexandimethanol
7,4 % Adipinsäure
8,4 % Isophthalsäure
2,9 % Dimethylolpropionsäure
9,3 % Isophorondiisocyanat
1,2 % Dimethylethanolamin
0,4 % ®Additol XW 395*
52,2 % Wasser

Dispersion IV

Wäßrige Dispersion eines Polyacrylats mit einem Festkörpergehalt von 45 % und einer OH-Zähl von ca. 60, hergestellt aus:
14,0 % Hydroxyethylmethacrylat
8,0 % Methylmethacrylat
18,3 % Butylacrylat
3,7 % Acrylsäure
1,5 % Azoisobuttersäurenitril
0,3 % t-Butyloctoat
2,0 % Ammoniak
52,2 % Wasser

Dispersion V

Wäßrige Dispersion eines Polyester-Polyurethans mit einem Festkörpergehalt von 45 % und einer OH-Zähl von ca. 33, hergestellt aus:
2,0 % Sojaölfettsäure
5,6 % Trimethylolpropan
10,1 % Hexandiol-1,6
7,1 % Adipinsäure
8,1 % Isophthalsärue
2,8 % Dimethylolpropionsäure
9,2 % Isophorondiisocyanat
0,1 % Zinn(II)oktoat
1,3 % Dimethylethanolamin
53,7 % Wasser

Dispersion VI

Wäßrige Dispersion eines Polyacrylats mit einem Festkörpergehalt von 40 % und einer OH-Zähl von ca. 38, hergestellt aus:
11,2 % Styrol
5,0 % Methylmethacrylat
13,5 % Butylacrylat
6,8 % Hydroxyethylmethacrylat
2,0 % Acrylsäure
0,7 % Dodecylmercaptan
0,3 % Dimethylethanolamin

*Handelsübliches Verlaufsmittel, Hersteller: Hoechst AG, Frankfurt

0,4 % Emulgator*
0,1 % Ammoniumperoxodisulfat
60,0 % Wasser

**Beispiele**

Beispiel 1

70,7 Teile Diisocyanat I und 29,3 Teile 2,2-Bis(hydroxymethyl)-propionsäure werden gemeinsam vorgelegt und auf ca. 80 °C erhitzt. Durch die einsetzende exotherme Reaktion steigt die Temperatur auf ca. 130 °C. Nachdem dieser Reaktionsschub abgeklungen ist, wird die Temperatur für 6 Stunden auf ca. 100 °C gehalten.

Man erhält einen farblosen Feststoff mit einem NCO-Gehalt von 7,6 %, einem Gehalt an Carboxylgruppen von 219 Milliäquivalenten pro 100 g und einem Schmelzpunkt von 135 bis 145 °C. Der Gehalt an monomerem Diisocyanat liegt bei 0,4 %.

Beispiel 2

33,2 Teile Diisocyanat I, 40,4 Teile des Polyesters und 6,4 Teile 2,2-Bis(hydroxymethyl)propionsäure werden in 20,0 Teilen Toluol vorgelegt. Man läßt zunächst eine Stunde bei 50 °C rühren, anschließend sieben Stunden bei 60 °C und weitere sieben Stunden bei 90 °C. Nach Abdestillieren des Lösungsmittels verbleibt ein farbloses wachsartiges Produkt mit einem NCO-Gehalt von 6,3 %, einem Gehalt an Carboxylgruppen von 60 Milliäquivalenten pro 100 g. Der Gehalt an monomerem Diisocyanat I liegt bei 1,4 %.

Beispiel3

50,0 Teile Diisocyanat I, 6,9 Teile 2,2-Bis(hydroxymethyl)propionsäure, 2,6 Teile Ethylenglykol und 5,5 Teile Trimethylolpropan werden in 35,0 Teilen Ethylacetat vorgelegt. Man läßt 18 Stunden bei 60 °C reagieren und erhält eine blaßgelbe Lösung mit einer Viskosität von 1 600 mPa.s (23 °C). Der Isocyanatgehalt beträgt 12,3 %, der Carboxylgruppengehalt 79 Milliäquivalenten pro 100 g und der Gehalt an monomerem Diisocyanat 1,1 %, jeweils bezogen auf Feststoff.

Zur Überprüfung der Lagerstabilität bei Raumtemperatur wurden der Gehalt an Isocyanatgruppen und die Viskosität über einen längeren Zeitraum verfolgt.

| Zeit (Monate) | NCO-Gehalt (%) | Viskosität (mPa.s 23 °C) |
|---|---|---|
| 0 | 8,0 | 1 600 |
| 2 | 7,8 | 1 800 |
| 4 | 7,7 | 1 950 |
| 6 | 7,7 | 2 200 |
| 8 | 7,6 | 2 300 |
| 10 | 7,5 | 2 450 |
| 12 | 7,4 | 6 600 |

Beispiel 4

50,0 Teile Diisocyanat I und 6,9 Teile 2,2-Bis(hydroxymethyl)propionsäure werden in 35,0 Teilen Butylacetat vorgelegt. Man läßt bei 50 °C 2,6 Teile Ethylenglykol und 5,5 Teile Trimethylolpropan gemeinsam aufgeschmolzen über zwei Stunden zutropfen. Es wird zunächst noch zwei Stunden bei derselben Temperatur und anschließend je acht Stunden bei 60 bzw. 100 °C nachgerührt. Die blaßgelbe Lösung weist eine Viskosität von 9 300 mPa.s (23 °C) auf. Die Isocyanatocarbonsäure weist einen NCO-Gehalt von 11,7 % einen Carboxylgruppengehalt von 79 MIlliäquivalenten pro 100 g und einen Gehalt an monomerem Diisocyanat von 0,2 %, jeweils bezogen auf Feststoff, auf.

*3-Benzyl-4-hydroxybiphenylpolyglykolether

Beispiel 5

51,1 Teile Diisocyanat I und 7,1 Teile 2,2-Bis(hydroxymethyl)propionsäure werden in 30,0 Teilen Diethylenglykoldimethylether vorgelegt und auf 50 °C erwärmt. 6,2 Teile 1,8-Octandiol und 5,6 Teile 1,2,6-Hexantriol werden gemeinsam in geschmolzenem Zustand über 1 Stunde zugetropft. Anschließend wird noch 12 Stunden bei 60 °C und 3 Stunden bei 80 °C nachgerührt. Die erhaltene Lösung weist eine Viskosität von 9 500 mPa.s (23 °C) auf. Die gelöste Isocyanatocarbonsäure weist einen NCO-Gehalt von 11,7 %, einen Carboxylgruppengehalt von 75 Milliäquivalenten pro 100 g und einen Gehalt an monomerem Diisocyanat von 1,1 %, jeweils bezogen auf Feststoff, auf.

Beispiel 6

36,0 Teile Diisocyanat I und 5,0 Teile 2,2-Bis(hydroxymethyl)-propionsäure werden in 30,0 Teilen Diethylenglykoldimethylether vorgelegt. Bei 50 °C läßt man 25,0 Teile des Polyesters und 4,0 Teile 1,2,6-Hexantriol gemeinsam als Schmelze über 2,5 Stunden zutropfen. Das Reaktionsgemisch wird anschließend 9 Stunden bei 60 °C und 3 Stunden bei 80 °C nachgerührt. Die resultierende Lösung weist eine Viskosität von 8 000 mPa.s (23 °C) auf. Die gelöste Isocyanatocarbonsäure weist einen NCO-Gehalt von 8,4 %, einen Carboxylgruppengehalt von 53 Milliäquivalenten pro 100 g und einen Gehalt an monomerem Diisocyanat von 1,3 %, jeweils bezogen auf Feststoff, auf.

Beispiel 7

48,5 Teile Diisocyanat I werden in 35,0 Teilen Ethylacetat vorgelegt. Bei 60 °C wird über vier Stunden ein Gemisch aus 5,1 Teilen 2,2,4-Trimethyl-1,3-pentandiol und 4,7 Teilen Trimethylolpropan zugetropft. Man läßt zehn Stunden bei 60 °C nachrühren und gibt 6,7 Teile 2,2-Bis(hydroxymethyl)propionsäure hinzu. Nach weiteren 18 Stunden bei 60 °C und acht Stunden bei 80 °C erhält man eine klare, blaßgelbe Lösung der Viskosität 900 mPa.s (23 °C). Die gelöste Isocyanatocarbonsäure weist einen NCO-Gehalt von 13,9 %, einen Carboxylgruppengehalt von 77 Milliäquivalenten pro 100 g und einen Gehalt an monomerem Diisocyanat von 1,5 %, jeweils bezogen auf Feststoff, auf.

Beispiel 8

52,5 Teile Diisocyanat I und 3,6 Teile 2,2-Bis(hydroxymethyl)-propionsäure werden in 30 Teilen Diethylenglykoldimethylether vorgelegt. Bei 55 °C werden 10,8 Teile 1,2,6-Hexantriol und 3,1 Teile Polyether I gemeinsam über drei Stunden zugetropft. Man läßt 12 Stunden bei derselben Temperatur und 8 Stunden bei 80 °C nachrühren. Die klare Lösung weist eine Viskosität von 1 600 mPa.s (23 °C) auf. Die gelöste Isocyanatocarbonsäure weist einen NCO-Gehalt von 14,3 %, einen Carboxylgruppengehalt von 39 Milliäquivalenten pro 100 g und einen Gehalt an monomerem Diisocyanat von 1,7 %, jeweils bezogen auf Feststoff, auf.

Beispiel 9

38,5 Teile Diisocyanat I und 2,7 Teile 2,2-Bis(hydroxymethyl)-propionsäure werden in 30,0 Teilen Diethylenglykoldimethylether vorgelegt. Bei 55 °C werden 22,3 Teile Polyether I, 4,0 Teile Trimethylolpropan und 2,5 Teile Ethylenglykol über acht Stunden zugetropft. Man läßt 12 Stunden bei derselben Temperatur und 12 Stunden bei 100 °C nachrühren. Die erhaltene Lösung weist eine Viskosität von 350 mPa.s (23 °C) auf. Die gelöste Isocyanatocarbonsäure weist einen NCO-Gehalt von 10,3 %, einen Carboxylgruppengehalt von 28 Milliäquivalenten pro 100 g und einen Gehalt an monomerem Diisocyanat von 1,8 %, jeweils bezogen auf Feststoff, auf.

Beispiel 10

48,6 Teile Diisocyanat I und 3,4 Teile 2,2-Bis(hydroxymethyl)-propionsäure werden in 30,0 Teilen Diethylenglykoldimethylether vorgelegt. Bei 55 °C werden 10,7 Teile 1,2,6-Hexantriol und 7,4 Teile Polyether II gemeinsam über sechs Stunden zugetropft. Man läßt 8 Stunden bei 60 °C, 8 Stunden bei 80 °C und 8 Stunden bei 100 °C nachrühren. Die klare gelbe Lösung weist eine Viskosität von 6 800 mPa.s (23 °C) auf. Die gelöste Isocyanatocarbonsäure weist einen NCO-Gehalt von 10,9 %, einen Carboxylgruppengehalt von 36 Milliäquivalenten pro 100 g und einen Gehalt an monomerem Diisocyanat von 0,9 %, jeweils

bezogen auf Feststoff, auf.

Beispiel 11

37,5 Teile Diisocyanat I und 2,6 Teile 2,2-Bis(hydroxymethyl)-propionsäure werden in 30,0 Teilen Diethylenglykoldimethylether vorgelegt. Hierzu tropft man bei 60 °C 21,7 Teile Polyether I und 8,2 Teile Trimethylolpropan gemeinsam über sechs Stunden zu. Nach weiteren 12 Stunden bei 60 °C und 12 Stunden bei 100 °C ist ein NCO-Gehalt von 6,0 % erreicht. Die Viskosität der Lösung beträgt 1 500 mPa.s (23 °C) auf. Die gelöste Isocyanatocarbonsäure weist einen NCO-Gehalt von 8,6 %, einen Carboxylgruppengehalt von 28 Milliäquivalenten pro 100 g und einen Gehalt an monomerem Diisocyanat von 1,3 %, jeweils bezogen auf Feststoff, auf.

Beispiel 12

46,3 Teile Diisocyanat I, 8,0 Teile Hexamethylendiisocyanat und 6,4 Teile 2,2-Bis(hydroxymethyl)-propionsäure werden in 30,0 Teilen Diethylenglykoldimethylether vorgelegt. Bei 50 °C wird über vier Stunden eine Schmelze aus 4,8 Teilen Trimethylolpropan und 4,5 Teilen Ethylenglykol zugetropft. Man läßt 14 Stunden bei 50 °C nachrühren und erhält eine klare farblose Lösung mit einer Viskosität von 4 500 mPa.s (23 °C). Die gelöste Isocyanatocarbonsäure weist einen NCO-Gehalt von 12,9 %, einen Carboxylgruppengehalt von 68 Milliäquivalenten pro 100 g, einen Gehalt an monomerem Diisocyanat I von 0,4 % und an monomerem Hexamethylendiisocyanat von 0,04 %, jeweils bezogen auf Feststoff, auf.

Beispiel 13

53,4 Teile Diisocyanat I und 5,5 Teile 2,2-Bis(hydroxymethyl)-propionsäure werden in 30,0 Teilen Diethylenglykoldimethylether vorgelegt. Bei 55 °C werden 11,1 Teile 1,2,6-Hexantriol über zwei Stunden zugetropft. Man läßt 12 Stunden bei 60 °C und 8 Stunden bei 80 °C nachrühren. Die klare Lösung weist eine Viskosität von 8 700 mPa.s (23 °C) auf. Die gelöste Isocyanatocarbonsäure weist einen NCO-Gehalt von 12,6 %, einen Carboxylgruppengehalt von 59 Milliäquivalenten pro 100 g und einen Gehalt an monomerem Diisocyanat von 0,9 %, jeweils bezogen auf Feststoff, auf.

Beispiel 14

49,5 Teile Diisocyanat I und 3,4 Teile 2,2-Bis(hydroxymethyl)-propionsäure werden in 30,0 Teilen Diethylenglykoldimethylether vorgelegt. Bei 50 °C wird eine aufgeschmolzene Mischung aus 11,3 Teilen Trimethylolpropan und 5,8 Teilen des Polyethers I über drei Stunden zugetropft. Es wird 15 Stunden bei 50 °C und 8 Stunden bei 75 °C nachgerührt. Die klare Lösung weist eine Viskosität von 9 500 mPa.s (23 °C) auf. Die gelöste Isocyanatocarbonsäure weist einen NCO-Gehalt von 11,9 %, einen Carboxylgruppengehalt von 37 Milliäquivalenten pro 100 g und einen Gehalt an monomerem Diisocyanat von 0,6 %, jeweils bezogen auf Feststoff, auf.

Beispiel 15

56,1 Teile Diisocyanat II, 6,4 Teile 2,2-Bis(hydroxymethyl)-propionsäure werden in 30,0 Teilen Diethylenglykoldimethylether vorgelegt. Bei 60 °C wird eine aufgeschmolzene Mischung aus 2,4 Teilen Ethylenglykol und 5,1 Teilen Trimethylolpropan über zwei Stunden zugetropft. Nach 12 Stunden bei 60 °C und 8 Stunden bei 80 °C Nachrührzeit erhält man eine blaßgelbe Lösung der Viskosität 7 600 mPa.s (23 °C) auf. Die gelöste Isocyanatocarbonsäure weist einen NCO-Gehalt von 9,9 %, einen Carboxylgruppengehalt von 68 Milliäquivalenten pro 100 g und einen Gehalt an monomerem Diisocyanat von 0,4 %, jeweils bezogen auf Feststoff, auf.

Beispiel 16

55,0 Teile Diisocyanat II und 3,1 Teile 2,2-Bis(hydroxymethyl)-propionsäure werden in 30,0 Teilen Diethylenglykoldimethylether vorgelegt und auf 60 °C erwärmt. Bei dieser Temperatur läßt man ein Gemisch aus 9,3 Teilen 1,2,6-Hexantriol und 2,6 Teilen Polyether I über drei Stunden zutropfen. Es wird 12 Stunden bei derselben Temperatur, 6 Stunden bei 80 °C und 1 Stunde bei 100 °C nachgerührt. Die erhaltene Lösung weist eine Viskosität von 1 750 mPa.s (23 °C) auf. Die gelöste Isocyanatocarbonsäure

weist einen NCO-Gehalt von 12,0 %, einen Carboxylgruppengehalt von 33 Milliäquivalenten pro 100 g und einen Gehalt an monomerem Diisocyanat von 1,6 %, jeweils bezogen auf Feststoff, auf.

Beispiel 17

50,7 Teile Diisocyanat II und 5,8 Teile 2,2-Bis(hydroxymethyl)-propionsäure werden in 35,0 Teilen Ethylacetat vorgelegt. Bei 55 °C läßt man ein Gemisch aus 4,4 Teilen 2,2,4-Trimethyl-1,3-pentandiol und 4,1 Teilen Trimethylolpropan über zwei Stunden zutropfen. Es wird 14 Stunden bei 60 °C und 8 Stunden bei 80 °C nachgerührt. Die erhaltene Lösung weist eine Viskosität von 1 100 mPa.s (23 °C) auf. Die gelöste Isocyanatocarbonsäure weist einen NCO-Gehalt von 11,9 %, einen Carboxylgruppengehalt von 66 Milliäquivalenten pro 100 g und einen Gehalt an monomerem Diisocyanat von 1,9 %, jeweils bezogen auf Feststoff, auf.

Beispiel 18 (Vergleichsbeispiel)

51,5 Teile Isophorondiisocyanat und 6,2 Teile 2,2-Bis(hydroxymethyl)-propionsäure werden in 35,0 Teilen Diethylenglykoldimethylether vorgelegt. Bei 50 °C werden 5,0 Teile Trimethylolpropan und 2,3 Teile Ethylenglykol über vier Stunden gemeinsam zugetropft. Man läßt 12 Stunden bei 100 °C nachrühren. Das klare Produkt weist einen Gehalt an Isocyanat von 7,5 % und an monomerem Diisocyanat von 4,4 % auf. Die Viskosität beträgt 76 000 mPa.s (23 °C).
Nach einer Lagerung über acht Wochen bei Raumtemperatur liegt der Gehalt an Isocyanat bei 7,0 % und die Viskosität bei 148 000 mPa.s (23 °C).

Beispiele zur Dispergierung der Polyisocyanatocarbonsäuren

Beispiel 19

21,5 Teile des in Beispiel 1 beschriebenen Produktes werden fein gemahlen und so in ein Gemisch aus 75 Teilen Wasser und 3,5 Teilen Dimethylethanolamin langsam eingerührt. Nach ca. zwanzigminütigem kräftigen Rühren entsteht eine homogene, dünnflüssige und blaustichige Dispersion. Die Eignung der in Wasser dispergierten Isocyanatocarbonsäuren als Vernetzer für H-aktive Bindemittelkomponenten bleibt während eines Zeitraums von ca. 6 Stunden praktisch unverändert.

Beispiel 20

Ein Gemisch aus 60,1 Teilen Wasser und 2,0 Teilen Dimethylethanolamin wird unter kräftigem Rühren zu 37,9 Teilen des Produktes aus Beispiel 2 gegeben. Nach ca. fünfminütigem weiteren Rühren entsteht eine homogene weiße Dispersion. Die Eignung der in Wasser dispergierten Isocyanatocarbonsäuren als Vernetzer für H-aktive Bindemittelkomponenten bleibt während eines Zeitraums von ca. 6 Stunden praktisch unverändert.

Beispiel 21

Ein Gemisch aus 47,4 Teilen Wasser und 2,3 Teilen Dimethylethanolamin wird unter kräftigem Rühren zu 50,3 Teilen der Polyisocyanatocarbonsäure aus Beispiel 3 gegeben. Hierbei entsteht eine feinteilige, dünne Dispersion, die schwach bläulich schimmert. Diese Dispersion ist über ca. acht Stunden stabil, d.h. erst nach dieser Zeit setzt eine merkliche Reaktion zwischen den Isocyanatgruppen und dem Wasser ein, die von einer Gasentwicklung begleitet wird.

Beispiel 22

48,3 Teile der in Beispiel 8 beschriebenen Polyisocyanatocarbonsäurelösung werden unter starkem Rühren in ein Gemisch aus 50,5 Teilen Wasser und 1,2 Teilen Dimethylethanolamin eingebracht. Es entsteht eine feinteilige Dispersion niedriger Viskosität, die leicht bläulich schimmert. Die Eignung der in Wasser dispergierten Isocyanatocarbonsäuren als Vernetzer für H-aktive Bindemittelkomponenten bleibt während eines Zeitraums von ca. 4 Stunden praktisch unverändert.

Beispiel 23

In 41,5 Teile des Produktes aus Beispiel 10 wird eine Mischung aus 57,6 Teilen Wasser und 0,9 Teilen Dimethylethanolamin eingerührt. Es entsteht zunächst eine relativ hochviskose Dispersion, die jedoch nach vollständiger Homogenisierung in eine dünnflüssige Dispersion mit ca. vier Stunden Topfzeit übergeht.

Beispiel 24

Eine Mischung aus 57,9 Teilen Wasser und 1,7 Teilen Dimethylethanolamin wird unter starkem Rühren in 40,4 Teile der Polyisocyanatocarbonsäurelösung aus Beispiel 12 eingetragen. Es entsteht eine feinteilige blaustichige Dispersion mit einer Verarbeitungszeit von ca. drei Stunden.

Beispiel 25

40,4 Teile des Produktes aus Beispiel 13 werden vorgelegt. Hierzu gibt man unter kräftigem Rühren ein Gemisch aus 57,9 Teilen Wasser und 1,7 Teilen Dimethylethanolamin. Nach der Homogenisierung liegt eine feinteilige Dispersion vor, die über ca. sechs Stunden nahezu unverändert vorliegt.

Beispiel 26

Ein Gemisch aus 54,7 Teilen Wasser und 1,5 Teilen Dimethylethanolamin wird unter kräftigem Rühren in 43,8 Teile des in Beispiel 13 beschriebenen Produkts eingerührt. Nach Homogenisierung entsteht eine niedrigviskose, feinteilige und schwach blaustichige Dispersion, die über mehrere Stunden nahezu unverändert stabil bleibt.

Beispiel 27

Zu 41,5 Teilen der Polyisocyanatocarbonsäure aus Beispiel 14 wird eine Mischung aus 57,4 Teilen Wasser und 1,0 Teilen Dimethylethanolamin eingerührt. Nach kurzzeitigem Weiterrühren entsteht eine homogene, feinteilige weißliche Dispersion. Diese Dispersion kann auch noch nach mehreren Stunden weiterverwendet werden.

Beispiel 28 (Vergleichsdispersion)

Beim Versuch, das Produkt aus Beispiel 18 analog Beispiel 19 zu dispergieren, kam es unter Gasentwicklung zur Bildung von Agglomeraten.

**Verwendung**

Zur Demonstration der erfindungsgemäßen Verwendung der erfindungsgemäßen Isocyanatocarbonsäuren wurden diese zur Vernetzung verschiedener hydroxylgruppenhaltiger wäßriger Polymerdispersionen herangezogen.

Die Vereinigung der Vernetzer mit den Polymerdispersionen erfolgte folgendermaßen: Die wäßrigen Polymerdispersionen wurden vorgelegt und mit 1 Gew.-% eines handelsüblichen Benetzungsmittels (Fluortensid FC 170 der Fa. 3M, Düsseldorf-Neuß, 10 %ige Lösung in Wasser) versetzt. Hierzu wurde das zur Neutralisation der Vernetzer benötigte Amin sowie weiteres Wasser gegeben und die gewünschte Menge der erfindungsgemäßen Isocyanatocarbonsäuren eingerührt. Die entstehenden zweikomponentigen wäßrigen Lackdispersionen weisen eine Verarbeitungszeit von ca. 4 bis 8 Stunden auf.

Die in Tabelle 1 beschriebenen Lackdispersionen wurden mit Spritzpistolen auf Glasplatten appliziert. Die Trocknung erfolgt über 20 Minuten bei 140 °C.

Die Bestimmung der Lösungsmittelbeständigkeit erfolgte durch eine 10- bzw. 1-minütige Einwirkung eines lösungsmittelgetränkten Wattebausches auf die Lackoberfläche (0 = Film unverändert, 5 = Film zerstört).

Tabelle 1

| Beispiel | 29 | 30 | 31 | 32 | 33 | 34 | 35 | 36 | 37 | 38 | 39 | 40 | 41 | 42 |
|---|---|---|---|---|---|---|---|---|---|---|---|---|---|---|
| OH-Dispersion | I | II | III | IV | II | IV | I | II | V | IV | VI | I | II | IV |
| Vernetzer | 13 | 13 | 13 | 13 | 8 | 8 | 14 | 14 | 14 | 14 | 14 | 14 | 14 | 14 |
| NCO/OH-Verhältnis | 1,2 | 1,2 | 1,2 | 1,2 | 1,2 | 1,2 | 1,2 | 1,2 | 1,2 | 1,2 | 1,2 | 1,7 | 1,7 | 1,7 |
| Festkörpergehalt [%] | 25 | 35 | 37 | 33 | 35 | 31 | 30 | 33 | 33 | 33 | 33 | 35 | 42 | 35 |
| Lösungsmittelgeh. [%] | 12 | 21 | 4 | 5 | 10 | 3 | 13 | 11 | 5 | 7 | 5 | 11 | 15 | 9 |
| Xylol 10' | 0 | 0 | 0 | 0 | 0 | 0 | 1 | 0 | 1 | 0 | 1 | 0 | 0 | 0 |
| Xylol/BuAc 1' *) | 0 | 0 | 0 | 0 | 0 | 0 | 0 | 0 | 0 | 0 | 0 | 0 | 0 | 0 |
| MPA/Aceton 1' *) | 0 | 0 | 0 | 0 | 1 | 0 | 1 | 0 | 1 | 0 | 1 | 1 | 0 | 0 |
| Erichsen-Tiefung [mm] DIN 53 156 | 10,0 | 3,5 | 4,0 | 0,5 | 10,5 | 2,5 | 10,5 | 10,5 | 8,0 | 2,5 | 6,0 | 10,0 | 8,0 | 1,5 |
| Pendelhärte [sec.] DIN 53 157 | 232 | 220 | 213 | 188 | 176 | 183 | 187 | 212 | 181 | 192 | 185 | 187 | 190 | 192 |

*)Gewichtsverhältnis: 1:1

Um die Wirksamkeit der erfindungsgemäßen Isocyanatocarbonsäurem zur Vernetzung wäßriger Polymerdispersionen zu unterstreichen, wurden zum Vergleich die verwendeten Polymerdispersionen auf Glasplatten appliziert, 20 min bei 140 °C eingebrannt (s. Tabelle 2) und denselben Test unterzogen. Aufgrund der schlechten Eigenschaften wurde jedoch auf die Messung der Erichsen-Tiefung verzichtet.

Tabelle 2

| (Vergleichsbeispiele) | | | | | | |
|---|---|---|---|---|---|---|
| Beispiele | 43 | 44 | 45 | 46 | 47 | 48 |
| OH-Dispersion | I | II | III | IV | V | VI |
| Xylol 10'<br>Xylol/BuAc 1'*<br>MPA/Aceton 1'* | 5<br>5<br>5 | 5<br>5<br>5 | 5<br>5<br>5 | 5<br>5<br>5 | 5<br>5<br>5 | 5<br>5<br>5 |
| Pendelhärte [sec] DIN 53 157 | klebriger Film | klebriger Film | 18 | 41 | klebriger Film | 76 |

*Gew.-Verhältnis: 1:1

**Patentansprüche**

**1.** Isocyanatocarbonsäuren, gekennzeichnet durch

a) einen Gehalt an, an (cyclo)aliphatische tertiäre Kohlenstoffatome gebundenen, Isocyanatgruppen von 1 bis 30 Gew.-% und

b) einen Gehalt an, gegebenenfalls zumindest teilweise in der Salzform vorliegenden, Carboxylgruppen von 0,5 bis 500 Milliäquivalenten pro 100 g.

**2.** Verfahren zur Herstellung von Isocyanatocarbonsäuren gemäß Anspruch 1, dadurch gekennzeichnet, daß man

A) Diisocyanate des Molekulargewichtsbereichs 168 bis 300 mit einer an ein primäres aliphatisches Kohlenstoffatom gebundenen Isocyanatgruppe und einer an ein tertiäres (cyclo)aliphatisches Kohlenstoffatom gebundenen Isocyanatgruppe, sowie gegebenenfalls

B) bis zu 25 NCO-Äquivalentprozent, bezogen auf die Gesamtmenge der Komponenten A) und B), an anderen Diisocyanaten des Molekulargewichtsbereichs 168 bis 300, mit (cyclo)aliphatisch gebundenen Isocyanatgruppen

mit

C) 2,2-Bis(hydroxymethyl)alkansäuren der Formel

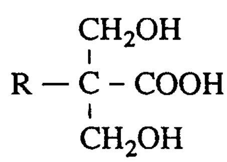

in welcher

R für Wasserstoff, einen Hydroxymethylrest oder einen Alkylrest mit bis zu 20 Kohlenstoffatomen steht,

sowie gegebenenfalls

D) sonstigen organischen Polyhydroxylverbindungen

unter Einhaltung eines NCO/OH-Äquivalentverhältnisses von 1,6:1 bis 2:1, gegebenenfalls unter vorhergehender oder anschließender zumindest teilweiser Neutralisation der Carboxylgruppen der Komponente C) mit einer Base, zur Reaktion bringt, wobei im übrigen Art und Mengenverhältnisse der Ausgangskomponenten so gewählt werden, daß die resultierenden Isocyanatocarbonsäuren den in Anspruch 1 genannten Angaben entsprechen.

**3.** Verfahren gemäß Anspruch 2, dadurch gekennzeichnet, daß man als Diisocyanate A) solche der Formel

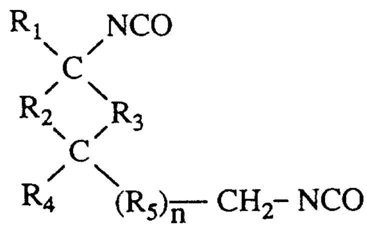

verwendet, wobei

| | |
|---|---|
| $R_1$ | für einen Alkylrest mit 1 bis 4 Kohlenstoffatomen, |
| $R_2$ und $R_3$ | für gleiche oder verschiedene zweiwertige, gesättigte aliphatische Kohlenwasserstoffreste mit 1 bis 4 Kohlenstoffatomen, |
| $R_4$ | für Wasserstoff oder einen Alkylrest mit 1 bis 4 Kohlenstoffatomen, |
| $R_5$ | für einen zweiwertigen, gesättigten aliphatischen Kohlenwasserstoffrest mit 1 bis 4 Kohlenstoffatomen und |
| n | für 0 oder 1 stehen. |

**4.** Verfahren gemäß Anspruch 2 und 3, dadurch gekennzeichnet, daß man als Komponente A) ein Diisocyanat ausgewählt aus der Gruppe bestehend aus 1-Isocyanato-1-methyl-4(3)-isocyanatomethylcyclohexan, 1-Isocyanato-1-methyl-4(4-isocyanatobut-2-yl)-cyclohexan und 1-Isocyanato-1,2,2-trimethyl-3-(2-isocyanatoethyl)-cyclopentan, verwendet.

**5.** Verfahren gemäß Anspruch 2 bis 4, dadurch gekennzeichnet, daß man als Komponente C) 2,2-Bis-(hydroxymethyl)propionsäure verwendet.

**6.** Verwendung der Isocyanatocarbonsäuren gemäß Anspruch 1 in zumindest teilweise neutralisierter Form als Vernetzer für wäßrige Beschichtungsmittel auf Basis von in Wasser gelöst und/oder dispergiert vorliegenden Kunststoffen oder Kunststoffvorläufern.

**Claims**

**1.** Isocyanatocarboxylic acids, characterised by

a) a content of 1 to 30 % by weight of isocyanate groups bonded to (cyclo)aliphatic tertiary carbon atoms and

b) a content of carboxyl groups, which are optionally present at least partly in salt form, of 0.5 to 500 milliequivalents per 100 g.

**2.** A method of preparing isocyanatocarboxylic acids according to claim 1, characterised in that

A) diisocyanates with a molecular weight range of 168 to 300, containing an isocyanate group bonded to a primary aliphatic carbon atom and an isocyanate group bonded to a tertiary (cyclo)-aliphatic carbon atom, and optionally

B) up to 25 equivalent percent of NCO, based on the total amount of components A) and B), of other diisocyanates with a molecular weight range of 168 to 300 containing (cyclo)aliphatically bonded isocyanate groups, are reacted

with

C) 2,2-bis-(hydroxymethyl)-alkanoic acids of formula

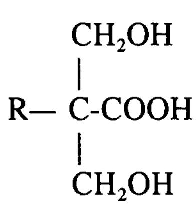

where

R denotes hydrogen, a hydroxymethyl radical or an alkyl radical having up to 20 carbon atoms,

and optionally with

D) other organic polyhydroxyl compounds

maintaining an NCO/OH equivalent ratio of 1.6:1 to 2:1, optionally with prior or subsequent at least partial neutralisation of the carboxyl groups of component C) with a base, wherein in addition the nature and quantitative ratios of the starting components are selected so that the resulting isocyanatocarboxylic acids correspond to the details given in claim 1.

**3.** A method according to claim 2, characterised in that diisocyanates of formula

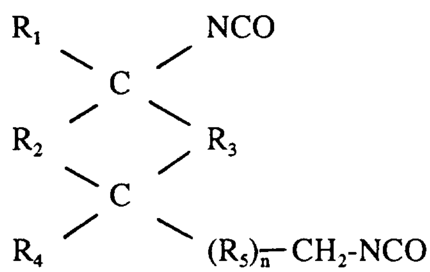

are used as diisocyanates A), where

| | |
|---|---|
| $R_1$ | denotes an alkyl radical having 1 to 4 carbon atoms, |
| $R_2$ and $R_3$ | denote identical or different divalent, saturated aliphatic hydrocarbon radicals having 1 to 4 carbon atoms, |
| $R_4$ | denotes hydrogen or an alkyl radical having 1 to 4 carbon atoms, |
| $R_5$ | denotes a divalent, saturated aliphatic hydrocarbon radical having 1 to 4 carbon atoms, and |
| n | denotes 0 or 1. |

**4.** A method according to claims 2 and 3, characterised in that a diisocyanate from the group consisting of 1-isocyanato-1-methyl-4(3)-isocyanatomethylcyclohexane, 1-isocyanato-1-methyl-4(4-isocyanatobut-2-yl)-cyclohexane and 1-isocyanato-1,2,2-trimethyl-3-(2-isocyanatoethyl)-cyclopentane is selected as component A).

**5.** A method according to claims 2 to 4, characterised in that 2,2-bis(hydroxymethyl)propionic acid is used as component C).

**6.** The use of the isocyanatocarboxylic acids according to claim 1 in at least partially neutralised form as cross-linking agents for aqueous coating media based on synthetic materials or synthetic material precursors which are present dissolved and/or dispersed in water.

## Revendications

**1.** Acides isocyanatocarboxyliques caractérisés par

a) une teneur en groupes isocyanate liés à des atomes de carbone tertiaires (cyclo)aliphatiques de 1 à 30% en poids et

b) une teneur en groupes carboxyle, éventuellement présents sous forme au moins partiellement saline, de 0,5 à 500 milliéquivalents par 100 g.

**2.** Procédé pour la préparation d'acides isocyanatocarboxyliques selon la revendication 1, caractérisé en ce qu'on amène à réagir

A) des diisocyanates du domaine de poids moléculaire de 168 à 300 contenant un groupe isocyanate lié à un atome de carbone aliphatique primaire et un groupe isocyanate lié à un atome de carbone (cyclo)aliphatique tertiaire, ainsi qu'éventuellement

B) jusqu'à concurrence de 25% d'équivalent NCO, rapportés à la quantité totale des composants A) et B) d'autres diisocyanates du domaine de poids moléculaire de 168 à 300 contenant des groupes isocyanate liés à des radicaux (cyclo)aliphatiques

avec

C) des acides 2,2-bis(hydroxyméthyl)alcanoïques répondant à la formule

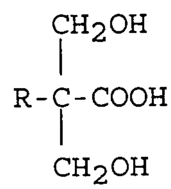

dans laquelle

R représente un atome d'hydrogène, un radical hydroxyméthyle ou un radical alkyle contenant jusqu'à 20 atomes de carbone,

ainsi qu'éventuellement

D) d'autres composés polyhydroxylés organiques,

en maintenant un rapport d'équivalents NCO/OH de 1,6:1 à 2:1, éventuellement avec neutralisation au moins partielle, préalable ou ultérieure, des groupes carboxyle du composant C) avec une base; dans lequel, par ailleurs, on choisit le type et les proportions des composants de départ de telle sorte que les acides isocyanatocarboxyliques résultants correspondent aux indications mentionnées à la revendication 1.

**3.** Procédé selon la revendication 2, caractérisé en ce qu'on utilise, comme diisocyanates A), ceux répondant à la formule

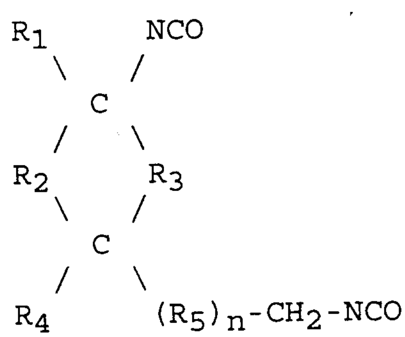

dans laquelle

$R_1$ représente un radical alkyle contenant de 1 à 4 atomes de carbone,

$R_2$ et $R_3$ représentent des radicaux d'hydrocarbures aliphatiques saturés bivalents, identiques ou différents, contenant de 1 à 4 atomes de carbone,

$R_4$ représente un atome d'hydrogène ou un radical alkyle contenant de 1 à 4 atomes de carbone,

$R_5$ représente un radical d'hydrocarbure aliphatique saturé bivalent contenant de 1 à 4 atomes de carbone et

n représente 0 ou 1.

**4.** Procédé selon les revendications 2 et 3, caractérisé en ce que, comme composant A), on utilise un diisocyanate choisi parmi le groupe constitué par le 1-isocyanato-1-méthyl-4(3)-isocyanatométhylcyclohexane, le 1-isocyanato-1-méthyl-4-(4-isocyanatobut-2-yl)-cyclohexane et le 1-isocyanato-1,2,2-triméthyl-3-(2-isocyanatoéthyl)-cyclopentane.

**5.** Procédé selon les revendications 2 à 4, caractérisé en ce que, comme composant C), on utilise l'acide 2,2-bis(hydroxyméthyl)propionique.

**6.** Utilisation des acides isocyanatocarboxyliques selon la revendication 1, sous forme au moins partiellement neutralisée, comme agents de réticulation pour des agents d'enduction aqueux à base de matières synthétiques ou de précurseurs de matières synthétiques présents sous forme dissoute et/ou dispersée dans l'eau.